# EUROPEAN PATENT APPLICATION

(11) **EP 3 320 972 A1**
(43) Date of publication of application: **16.05.2018**
(21) Application number: 16382519.3
(22) Date of filing: 09.11.2016
(51) Int. Cl.: B01J 27/043, C02F 1/72, A61L 2/232, B01J 27/057, B01J 21/18, B01J 37/03, B01J 21/06, C02F 1/78, C02F 1/74, B01J 27/20, C02F 1/50, B01J 37/02, C02F 103/00

(54) **HOUSEHOLD APPLIANCE HAVING A SELF-CLEANING CATALYTICALLY ACTIVE SURFACE AND A METHOD FOR OPERATING THE SAME**

(71) Applicant: BSH Hausgeräte GmbH, 81739 München (DE); BSH Electrodomésticos España, S.A., 50016 Zaragoza (ES)
(72) Inventor: Artal Lahoz, Maria Carmen, 50007 Zaragoza (ES); Bischof, Andreas, 10407 Berlin (DE); Bunuel Magdalena, Miguel Angel, 50017 Zaragoza (ES); Lazaro Villarroya, Guillermo Alberto, 50005 Zaragoza (ES); Sanz Naval, Javier, 50193 Zaragoza (ES)

(57) **Abstract**

The invention relates to a A household appliance (1) with at least one self-cleaning catalytically active surface containing at least one reactive oxygen species (ROS) generator (17), wherein the at least one ROS generator is selected from the group consisting of compounds with the formula (I), (II) or (III),

Ag-B-M-Sulfur (I)

wherein
B is selected from the group consisting of Al, Ga, In, Sn, Tl, Pb and Bi; and
M is selected from the group consisting of Ti, Zr, Cr, Mo, Mn, Fe, Ru, Ni, Pd, Cu, Zn and Cd; in particular the compound Ag-In-Ni-Sulfur;

MO_{q}-A (II)

wherein
q = 2 or 3; and
M is selected from the group consisting of Zr, Ti, Cr, Mo, W and Mn; and
A is acetylacetonate or a derivative thereof selected from the group consisting of compounds with the formula: 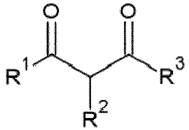 wherein
R¹, R² and R³ are independently selected from the group consisting of H, Cl, Br, I, and C₁ to C₈₀ hydrocarbon and polymer radicals.

YSe-Rₓ-MO₂ (III)

wherein
Y is selected from Sn or Pb; and
Rₓ is a carbon allotrope; and
M is selected from the group consisting of Zr, Ti, Cr, Mo, W and Mn.

## Description

The invention relates to a household appliance with at least one self-cleaning catalytically active surface containing at least one reactive oxygen species (ROS) generator and a method for operating the same.

Household appliances can come into contact with dust, dirt, food, humidity or human skin.
This may lead to hygienic problems in the long term, since microorganisms may deposit on the household appliance and may proliferate.

Moreover, food stains are common in household appliances where they produce a number of problems. In general, the presence of stains on the external parts is related to a bad hygiene feeling. It is difficult to keep clean some surfaces as for example such of stainless steel and glass. Some stains, for example burnt ones, need a great effort of cleaning by consumers. Food stains are perceived in cooking scenarios (hobs, ovens), but also in storage home appliances as for example refrigerators.

In addition, articles which have been soiled in various ways are in general cleaned in water-bearing household appliances. Food remains therefore arise in dishwashers and the range of dirt occurring in laundry items to be cleaned in washing machines is typically even greater. All water-bearing household appliances have in common that in the humid and warm atmosphere, in particular at sites that are difficult to access, dirt can arise and accumulate. This dirt may be a good nutrient medium for microorganisms such as bacteria and fungi.

A hygiene problem exists, particularly where water remains in a household appliance. Therefore, in a laundry treatment device, for example, a washing machine, when a water reservoir is used or on frequent use of washing programs with cold liquors, during long phases of non-use with the door closed or under unfavorable placing conditions, dirt can arise, particularly the formation of biofilms comprising organic substances such as microorganisms and nutrients. Said biofilms lead to bad odors and/or visible dirt. Pathological effects may even occur. Biofilms form particularly in areas of washing machines in which water is present for long periods, i.e. during phases of non-use. Such areas include poorly ventilated areas such as ribs, pockets, folds in seals, water inflows and outflows and, most particularly, water reservoirs which serve to store the greywater arising in the washing and rinsing process for re-use in a later washing or rinsing process. It is therefore desirable to sanitize the water in a household appliance and to prevent the formation of biofilms.

Other household appliances that may tend to contaminations with microorganisms are for example kitchen hoods or vacuum cleaners.

Irrespective of the type of the household appliance, a problem arises in that for controlling the household appliance an operating panel must be touched in general by a user. In this way operating panels can be contaminated with microorganisms which may moreover be spread when the household appliance is used by several persons.

In order to maintain a hygienically impeccable state in a household appliance various methods are currently being used. In particular, various measures for removing and/or preventing biofilms are known from the prior art. In water-bearing household appliances, sometimes machine cleaning programs are offered which remove built-up of dirt at high temperatures with the assistance of washing agents and sometimes with increased liquor levels and/or at raised drum rotation speeds, i.e. with an increased input of mechanical energy. Also known is the use of ozone to remove organic dirt.

Among the best known methods, the use of antimicrobial active ingredients in the surface, for example silver ions, is envisaged. These have however the disadvantage that they are gradually removed from the surface and are thus depleted. Moreover, when Ag+ or Cu+ ions are used in water-bearing household appliances, for example in the washing liquor and on the surfaces of the materials coming into contact with the washing liquor, detrimental effects with regard to groundwater and waterway pollution also arise. However, if these active ingredients are not washed out from a carrier material in the surfaces of the household appliance, their efficacy is mostly small such that over a long term microorganisms and even biofilms are being formed on the surface and negatively affect the hygienic state of the household appliance.

It has also previously been proposed to remove organic dirt with the aid of UV-C-radiators using a through-flow principle, the removal taking place in such a manner that microorganisms in such dirt are finally killed by damaging their genetic material.

Other methods are directed to thermal killing of any microorganisms present by increasing the temperature at the surfaces of the components coming into contact with the washing liquor, via direct or indirect energy transfer (water, steam, microwaves).

Photocatalytic methods are also known, for example, the use of catalytically effective compounds, in particular titanium dioxide coatings for deodorizing, disinfecting and cleaning. In this process, the catalyst needs to be often activated by means of UV radiation. These compounds support the oxidative modification or destruction of microorganisms, such that they are removed in the best case in total by oxidation. Often this method works, however, only in a limited manner, in particular if UV light is required in addition for the oxidation.

A disadvantage of these known methods and measures is the high energy usage and the sometimes high apparatus and/or operating costs for achieving remarkable effects. In some methods, potentially health-endangering agents, for example ozone or UV radiation, are used so that additional safety measures are required.

Specific surface structures which make use of the lotus effect aim at reducing the adhesion of dirt and at easing the cleaning of the surfaces. There is in general no antimicrobial effect of these surfaces. A big disadvantage of all these methods is that they all operate very locally. Thus, there is no long range antimicrobial effect in the free water of a water storage container.

Another method involves the formation of reactive oxygen species (ROS) as oxidants to remove and/or prevent the growth of microorganisms and the formation of biofilms. ROS generators currently applied in household appliances are polyoxometalate compounds. Polyoxometalates were found to be capable of oxidizing some organic substances and to generate reactive oxygen species (ROS) as efficient oxidants during regeneration of the reduced polyoxometalate. However, a major drawback associated with the use of polyoxometalates is that ROS, which provide the main catalytic effect in preventing and removing biofilms, are generated at a comparably low rate only. The concentration of ROS is high in direct vicinity to the polyoxometalate source, but the concentration gradient rapidly declines with increasing distance from the polyoxometalate source. Furthermore, the low ROS generation rate also affects the time of use of a polyoxometalate as catalyst, the lifetime of which should be as long as possible.

The use of polyoxometalates as ROS generators in household appliances is known.

The publication EP 1 141 210 B1 discloses a method for bleaching laundry items or household surfaces wherein a washing agent which contains polyoxometalates is brought into contact with the soiled substrate. Air serves as the primary source of oxygen atoms for bleaching.

The publication DE 10 2009 026 712 A1 discloses a household appliance having at least one component which has a surface that can be affected by organic dirt, said surface having a photocatalyst, a photosource for irradiating the photocatalyst with an activating electromagnetic radiation being associated with said surface, the surface being formed from a primary formed first material in which the photocatalyst is dispersed. Materials with titanium dioxide and modifications thereof are disclosed in great detail as photocatalysts.

The publications EP 2 761 073 B1 and US2014/231363 A1 disclose a water-bearing domestic appliance having a container for receiving objects to be cleaned and at least one inner surface containing a catalytically active substance, said surface being disposed inside the domestic appliance, wherein the catalytically active substance is a polyoxometalate and the inner surface comes into contact with water to be cleaned during operation of the domestic appliance. Preferably the polyoxometalate is tungstate and even more preferably the tungstate is modified by titanium.

The publication WO 2015/0787737 A1 discloses a water-conducting domestic appliance having a storage tank for storing a quantity of gray water and a treatment device for treating the gray water stored in the storage tank. The water-conducting domestic appliance is configured for determining the quantity of gray water stored in the storage tank and for operating the treatment device subject to the determined quantity of gray water stored in the storage tank.

The publications DE 10 2013 205 302 A1 and WO 2014/154432 A1 disclose a household appliance which comprises at least one catalytically effective substance in a surface, wherein the catalytically effective substance is a polyoxometalate that is comprised in an inner and/or outer surface of the household appliance, provided that the polyoxometalate is comprised at least in an outer surface of the household appliance if the household appliance is a water-bearing household appliance having a container for receiving objects to be cleaned.

The publication WO 2014/122225 A1 discloses the use of a heteropolyoxometalate of the Formula (I), (II) or (III)

A_{q+3}PV_{q}Z_{12-q}O₄₀ (I),

A₆P₂Z₁₈O₆₂ (II),

or

A₃PZ₄O₂₄ (III)

wherein Z is selected from Mo or W,
index q = 0, 1, 2 or 3, and
A is selected from one or more cations and comprises at least one cation selected from the group consisting of quaternary ammonium cations, quaternary phosphonium cations and tertiary sulfonium cations for providing self-cleaning, stripping, disinfecting, self-sanitizing, biocidal, antimicrobial, and/or deodorizing properties to at least part of a substrate or a surface of a substrate or to a coating or for decomposition and/or degradation of organic materials. In Formula (I), Z is preferably Mo and q = 2, and in Formula (III), Z is preferably W.

The publication EP 2 765 136 A1 discloses a heteropolyoxometalate of the Formula (I), (II) or (III)

A_{q+3}PV_{q}Z₁₂₋qO₄₀ (I),

A₆P₂Z₁₈O₆₂ (II),

or

A₃PZ₄O₂₄ (III)

wherein Z is selected from Mo or W,
q is 1, 2 or 3, and
A is selected among one or more cations and comprises at least one quaternary ammonium cation with the proviso that the compounds [(n-C₄H₉)₄N]₃PMo₁₂O₄₀ and [(n-C₆H₉)₄N]₃PMo₁₂O₄₀ are exempted. Most preferred are the heteropolyoxometalates [(n-C₄H₉)₄N]₃PW₄O₂₄ and [(n-C₆H₁₃)₄N]₃PW₄O₂₄.

To the best of our knowledge the use of other ROS generators than polyoxometalates in household appliances is not known.

In view of this situation, an object underlying the present invention is to provide a household appliance and a method for the operation thereof providing an improvement on removing or avoiding or at least vastly removing or avoiding hygiene affecting contaminations.

This object is achieved according to the invention with a household appliance and a method for operating the same, having the features of the corresponding independent claims. Preferred embodiments of the household appliance according to the invention are disclosed in the corresponding dependent claims. Preferred embodiments of the household appliance according to the invention correspond to preferred embodiments of the method according to the invention and vice versa, even if not explicitly stated herein.

The invention is thus directed to a household appliance with at least one self-cleaning catalytically active surface containing at least one reactive oxygen species (ROS) generator, wherein the at least one ROS generator is selected from the group consisting of compounds with the formula (I), (II) or (III);

**Ag-B-M-Sulfur** **(I)**

wherein
B is selected from the group consisting of Al, Ga, In, Sn, Tl, Pb and Bi; and
M is selected from the group consisting of Ti, Zr, Cr, Mo, Mn, Fe, Ru, Ni, Pd, Cu, Zn and Cd;
**MO_{q}-A** **(II)**

wherein
q = 2 or 3; and
M is selected from the group consisting of Zr, Ti, Cr, Mo, W and Mn; and
A is acetylacetonate or a derivative thereof selected from the group consisting of compounds with the formula: 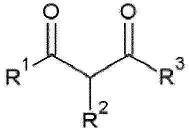 wherein
R¹, R² and R³ are independently selected from the group consisting of H, Cl, Br, I, and C₁ to C₈₀ hydrocarbon and polymer radicals;

**YSe-Rₓ-MO₂** **(III)**

wherein
Y is selected from Sn or Pb; and
Rₓ is a carbon allotrope; and
M is selected from the group consisting of Zr, Ti, Cr, Mo, W and Mn.

A household appliance is preferred, wherein the at least one ROS generator is

**Ag-In-Ni-Sulfur** **(I')**

In a preferred embodiment, the Ag-In-Ni-Sulfur compound is used in the form of a nanocomposite. The nanocomposite may generally be prepared in any suitable way, e.g. hydrothermally.

In a preferred embodiment, the above compound is prepared by dissolving silver acetate, indium acetate, nickel tetrahydrate and sodium dodecyl sulfate in water to prepare a solution A. Separate thereof, thioacetamide is dissolved in water to prepare a solution B. Both solutions A and B are transferred into an autoclave, for example a 25 mL Teflon lined sealed stainless steel autoclave, and heated, for example by placing the solutions A and B in a preheated oven at 130 °C for 2h before allowing to cool to room temperature. A black solid precipitate is then in general obtained by centrifugation and followed by washing with deionized water and ethanol and finally drying under vacuum for 2h.

In a specific example, the above compound is prepared in that silver acetate (1.0 mmol), indium acetate (1.0 mmol), nickel acetate tetrahydrate (1.0 mmol) and sodium dodecyl sulfate (0.1-1.15 mmol) are dissolved in 10 mL water (solution A). In a separate flask, thioacetamide (3.5 mmol) is dissolved in 5 mL of water (solution B).

Another household appliance is preferred, wherein the at least one ROS generator is

**ZrO₂-A** **(II')**

wherein A is acetylacetonate.

In a preferred embodiment, the above ZrO₂-A compound is prepared in a sol-gel process. In this way, the compound can be obtained as porous amorphous material after drying the gel at room temperature.

Preferably the above compound is obtainable by preparing a first solution containing zirconium (IV) propoxid, acetylacetone and 1-propanol, and by adding thereto a second solution containing water and 1-propanol. It is then in general stirred, preferably at a temperature from 20 to 30°C, until gelation and the obtained gel is then dried to obtain an amorphous product.

In a preferred example the above compound is obtainable by preparing and stirring at room temperature a first solution containing zirconium (IV) propoxid (70wt% in 1-propanol, 10 mL, 22.6 mmol), acetylacetone (99+%, 1.5 mL, 14.6 mmol) and 1-propanol (99.80+%, 3.0 mL, 39.9 mmol). A second solution containing distilled water (3.0 mL, 166 mmol) and 1-propanol (5.5 mL, 73.2 mmol) is added to the first solution. The solution obtained is then vigorously stirred for about 20 min at room temperature, until gelation occurs. A homogeneously slightly yellow colored gel is obtained. The gel is left at room temperature for 24h and then dried at 30°C to obtain the porous amorphous material.

Furthermore, a household appliance is also preferred, wherein the at least one ROS generator is

**PbSe-Cx-TiO₂** **(III')**

wherein Cₓ is graphene.

The above compound may be prepared in any suitable way. In a preferred embodiment, the above compound is obtainable by mixing graphene oxide and PbCl₂ to obtain a solution A. A solution B is also prepared by dissolving Na₂SO₃ and selenium powder in water and forming Na₂SeSO₃ under reflux. Solution B and aqueous ammonia are then added to solution A, followed by heating to a temperature in the range of 50 to 80°C. A colloidal aqueous TiO₂ solution (solution C) is then added to the mixture and finally the suspension is sonicated at room temperature. Thereafter, the reaction mixture is allowed to cool to room temperature, washed with water and dried to obtain the desired compound.

In a preferred example, the above compound is obtainable by dispersing 300 mg graphene oxide and 1.76 g PbCl₂ in 80 mL ethylene glycol by ultrasonication for 1h to obtain even graphene oxide nanosheets (GONS)/Pb²⁺ (solution A). In addition, Na₂SO₃ (5g) and selenium powder are dissolved in 30 mL distilled water and refluxed for 1h to form Na₂SeSO₃ (solution B). Solution B and 6 mL NH₄OH (28 wt.%) are added to solution A, followed by heating to 60°C for several minutes. A colloidal TiO₂ solution (solution C) with the molar ratios of ethanol:H₂O:TNB = 35:15:4 is added to the mixture and finally the suspension is sonicated at room temperature for 0, 1, 3, 5h using a controllable serial-ultrasonic apparatus. The reaction mixture is allowed to cool to room temperature and the precipitate is filtered, washed with distilled water, and dried in a vacuum oven at 80°C for 12h before heat treatment at 500°C for 1 h.

The self-cleaning catalytically active surface may in general be obtained during the manufacture of the household appliance of the present invention.

In a preferred embodiment, the self-cleaning catalytically active surface is obtainable by admixing at least one ROS generator with the surface forming material prior to or during its manufacture.

In a preferred embodiment of the household appliance the self-cleaning catalytically active surface is provided on a polymer body. The at least one ROS generator can then be mixed with the polymeric material in order to obtain a dispersion or a compound of ROS generator and polymer. Said dispersion or compound can e.g. be submitted to injection molding or extrusion for forming the polymer body. The polymeric material can be a thermoplastic elastomer, a duroplast or an elastomer. In particular, polyacrylates, acrylonitrile-butadiene-rubber, polytetrafluoroethylene, silicone, acrylate resins, polyurethane resins, silicone resins, polyester resins, alkyd resins, epoxy resins, phenolic resins, and urea or amine based resins or a mixture therefore are preferable.

Alternatively, the at least one ROS generator may also be mixed with monomers before polymerization. After polymerization, the resulting ROS generator modified polymer can then be submitted to, for example, compounding processes, pressureless processing techniques like casting or foaming, or compression molding, rolling and calendaring, extrusion, blow molding or injection molding processes or drawing, thermoforming or printing for forming the polymer body.

In another preferred embodiment of the invention, the self-cleaning catalytically active surface is obtainable by generating a layer containing at least one ROS generator on the surface providing item.

Such a layer may be formed in various ways, for example via coating or the application of paint or varnish containing the at least one ROS generator. Also the formation of multiple layers is possible.

In a preferred embodiment, however, the at least one ROS generator is applied together with a polymeric organic or inorganic binder. The binder may preferably be selected from among acrylate-based binders, polyurethane-based binders and silicone-based binders.

Suitable acrylate-based binders are for example Gräsolin 2K-Acryllack from the company Gräsolin, WorléeCryl A1220 from the company Worlée Chemie, Bayhydrol® A145 from the company Covestro, a water-reducible, hydroxyfunctional polyacrylic dispersion, and Desmophen® A265BA from the company Covestro.

A suitable polyurethane (PUR)-based binder is for example ATCOAT Atrepur 340 from the company ATCOAT.

Suitable silicone resin-based binders are for example Silikopon® EF from the company Evonik, 2577 Low Voc from the company Dow Corning or Bluesil RES 991 from the company Bluesil.

In this way, in the production of a household appliance, the component to be provided with a self-cleaning catalytically active surface containing at least one ROS generator may hen be obtained for example by dip-coating the component into a liquid containing the binder and the at least one ROS generator.

Moreover, it is possible to produce a foil wherein the at least one ROS generator is combined with a suitable carrier material and the binder, such that only a comparatively small amount of the at least one ROS generator might be required in order to cover a large surface area. Such foils can be applied to relevant parts in the household appliance, for example by glueing or meltbonding. Moreover, such foils may be placed directly in an injection mould and thus directly utilized for the manufacture of parts of the household appliance.

Said self-cleaning catalytically active surface can also be created by placing particles of the at least one ROS generator at the surface of a porous material using a suitable binder. The production thereof generally depends on the location of the surface and the manner in which the household appliance is being used.

In another preferred embodiment, the self-cleaning catalytically active surface may furthermore also contain a mixture of different ROS generators selected from the group consisting of compounds with the formula (I), (II) or (III). It is thus possible, that e.g. one or more ROS generator compounds are admixed with the surface forming material prior to or during its manufacture and/or that one or more ROS generator compounds are applied in a layer on said manufactured surface. In this way, synergistic effects among different ROS generator compounds can be exploited.

Generally, a dissolution into a surrounding liquid medium or any other depletion of the at least one ROS generator from the surface should be avoided. In a preferred embodiment, thus for ROS generators according to formula (II), A is a derivative of acetylacetonate selected from the group consisting of compounds with the formula: 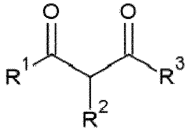 wherein R¹, R² and R³ are independently selected from the group preferably consisting of H, Cl, Br, I, and C₁ to C₈₀ hydrocarbon and polymer radicals. Even more preferably R¹ and R³ are independently selected from the group consisting of C₂₀ to C₈₀ hydrocarbon and polymer radicals, and R² is selected from the group consisting of C₁ to C₁₀ hydrocarbon radicals. It has been found that especially longer chain hydrocarbons help to better embed the ROS generator compounds into the matrix of a surface.

The household appliance is not limited. However, the household appliance according to a preferred embodiment of the invention is a water-bearing household appliance. In general, a water-bearing household appliance is a household appliance during the operation of which water is used. The items to be cleaned can be, in particular, tableware or laundry items. Cleaning should according to the invention also be understood to mean freshening. Accordingly, a water-bearing household appliance can be also a dryer.

In the case of a water-bearing household appliance it is preferable that the surface is an interior surface provided in the interior of the household appliance which comes into contact with flowing or static water during operation of the household appliance. The contact is usually necessary, since organic substances in the water of the household appliance are oxidized due to the contact with the at least one ROS generator-containing surface and are thereby decomposed.

In the case of a water-bearing household appliance it is preferable that at least one interior surface is provided in a water feed system. In addition or alternatively it is preferable that the at least one interior surface is provided in a water reservoir. It is then advantageous that the water reservoir comprises a circulating element and/or an air infeed element.

In a preferred embodiment, the water-bearing household appliance is a dishwasher or a laundry treatment device or a coffee making machine.

In another preferred embodiment, the water-bearing household appliance is a laundry treatment device belonging to the group consisting of a washing machine and a washer-dryer.

The inventive household appliance enables for example the water in a water-bearing household appliance to be treated in an efficient, economical and environmentally-friendly way such that organic substances like microorganisms and nutrients are decomposed and the formation of a biofilm in the household appliance can be avoided. In the present invention, the property of the at least one ROS generator, and in particular of the specific ROS generators described herein, which do not need an activation by radiation and also work under the exclusion of light, as oxidation catalyst or as oxidation agent is utilized.

In the oxidation reaction, the ROS generator serves, in general, as an oxidation catalyst which cooperates with an oxidizing agent. The oxidizing agent is not limited according to the invention. Preferably, an oxygen-containing oxidizing agent is used. Oxygen, inorganic or organic peroxides and/or ozone are particularly preferred as oxidizing agents. Oxygen is again particularly preferred amongst them as an oxidizing agent since an additional input of possibly harmful or interfering substances can be avoided. Air is preferably used as a source of oxygen. However, the oxygen may be present, for example, dissolved in water. Furthermore, by means of a corresponding movement of the water, mixing of air and water can take place, for example, for the formation of air bubbles in the water and an air-water mixture of this type can be brought into contact with the at least one ROS generator containing surface, the atmospheric oxygen preferably serving as the oxidizing agent. Particularly preferably, an air infeed element provides a sufficient quantity of oxygen in the water to be treated.

The ROS generators discussed herein can have antimicrobial properties due their flexible redox behavior. Namely, the ROS generator can act as both oxidizing and reducing agent depending on the type of reaction. After the reduction of the ROS generator, e.g. after contact with bacteria or fungi, its reduced form can be regenerated by oxygen. Super-oxide ions O₂^{-.} are generated as secondary species during this process. The super-oxide ions are also very efficient oxidants. Moreover, OH-radicals can be formed as a further strong oxidant.

In another embodiment, a peroxide or ozone is used as the oxidizing agent.

Ozone may be preferred as an oxidizing agent in a household appliance having an ozone generator. Such a household appliance preferably has also an ozone removal device. The at least one ROS generator-containing surface is then arranged as such that said surface can come into contact with water and ozone. For example, in a washing machine in which ozone can be introduced into the outer tub, the at least one ROS generator-containing surface is arranged in the outer tub accordingly.

If a peroxide is used as the oxidizing agent, preferably peroxide already present in the laundry care product is used. In this manner, the addition of further peroxide can be avoided.

In a preferred household appliance of the present invention, in particular a water-bearing household appliance, and more particularly a washing machine or a wash-dryer, the self-cleaning catalytically active surface containing at least one ROS generator is arranged in the detergent dispenser tray or in the outer tub, particularly preferably in the outer tub if peroxides serve as the oxidizing agent. The reason therefore is that peroxides are present particularly in heavy-duty washing agents and bleaching agents, although bleaching agents in particular are often added directly in the drum.

Preferably, the user of the laundry treatment device according to an embodiment of the household appliance of the present invention can specify, by means of selection buttons, whether heavy-duty washing agents or bleaching agents have been added. Alternatively, the presence of strong oxidizing agents such as peroxides or ozone can be established by means of one or more sensors in the washing machine. In the presence of ozone or peroxides, the operation of a circulating element or an air infeed element can be dispensed with.

In another preferred household appliance of the present invention the self cleaning catalytically active surface containing the at least one ROS generator may be provided in the water reservoir of an aerosol generator. In this way, reactive oxygen species generated in the water reservoir can be distributed throughout the household appliance in the form of an aerosol preferably carried by an air stream.

As discussed above, in the presence of an oxidizing agent such as oxygen, hydrogen peroxide or ozone, oxygen radicals (or oxygen-containing radicals, herein referred altogether as "oxygen radicals") form at the self-cleaning at least one ROS generator-containing surface of the household appliance, irrespective of whether it is an internal or external surface. When these highly reactive oxygen radicals come into contact with organic substances including microorganisms, an oxidation reaction takes place which leads to the decomposition of the organic substances and/or to pathological processes in the microorganisms. In this way, the formation of a biofilm is counteracted. For example organic substances in water which is brought into contact with the interior surface of a water-bearing household appliance coated with at least one of the ROS generator compounds described herein are decomposed and therefore the formation of a biofilm can be avoided.

In order to enable a continuous oxidation reaction, a sufficient level of mobility of the oxidizing species, for example, of oxygen radicals, which have been generated at the at least one ROS generator-containing surface acting as an oxidation catalyst is preferable. This could be for example achieved by recirculating an aqueous medium with the aid of a pump. In cases where there is in general no flow around the at least one ROS generator-containing surface, it might be sufficient to exploit natural diffusion, or convection in case of temperature differences. It might be possible to dispense with a sufficient mobility at the surface when the desired disinfection is to be achieved first of all at a surface itself, and where a long range effect, for example a complete disinfection of the surrounding medium, by means of reactive oxygen species can be dispensed with. This is true especially in the case of control panels which should be in a hygienic state as such.

Furthermore, a sufficiently high concentration of oxidizing agent should be present in the water to be treated in order to achieve a sufficient hygienic effect.

In the household appliance of the present invention it is preferred that the self-cleaning catalytically active surface has an at least one ROS generator containing upper layer with a thickness of from 0.01 to 1.5 mm, preferably a thickness of from 0.01 to 0.5 mm, more preferably of from 0.02 to 1 mm, and even more preferably of from 0.05 to 0.5 mm.

When the at least one ROS generator is used together with an acrylate-based binder, a polyurethane-based binder or a silicone resin-based binder, the coating thickness is preferably in the range of from 10 nm to 500 µm and more preferably in the range of from 20 nm to 200 µm.

It is moreover preferred that in the household appliance the upper layer of the surface has a content of the at least one ROS generator in the range of from 0.1 to 90 wt%, more preferably in the range of from 0.5 to 50 wt%, and even more preferably in the range of from 1 to 10 wt%, based on the weight of the upper layer (in the following also to be termed "surface layer").

In the household appliance according to the invention, in principle any surface can contain at least one ROS generator. These surfaces can be interior or exterior surfaces.

The at least one ROS generator-containing surfaces may be outer surfaces of handles, knobs, handle bars, controls, or of switches, for example light switches, handrails, sliders, control dials, slide controls ortouchsensitive (sensor active) surfaces.

In a dryer as a household appliance according to the present invention, the surface can be for example the container accepting items to be dried and the condensate container. In general all water-bearing parts in a household appliance, for example pumps including their housing, tubing, valves can contain at least one ROS generator-containing surfaces.

An interior at least one ROS generator containing surface might be an inner wall of a water container and/or of a flow-through part of a water-bearing household appliance.

The water container can be for example the water container of a coffee percolator. Moreover, all other parts of a percolator coming into contact with water or milk etc. can have at least one ROS generator-containing self-cleaning surfaces.

Household appliances according to the present invention include moreover containers wherein waste, garbage or used, spoiled food is temporarily stored.

Other uses of the invention are for the disinfection of the surfaces in scales or balances, in order to avoid the transfer of pathogenic microorganisms. The latter are thus also household appliances according to the present invention.

In the case where the household appliance is a washing machine, it is especially advantageous if the outer tub has surfaces that contain at least one ROS generator. The formation of biofilms is avoided and moreover leaching may be activated in the course of a washing program. Another part in a washing machine is the detergent dispenser tray wherein also biofilm formation can be avoided.

In a dishwasher as household appliance of the present invention, an at least one ROS generator containing surface may assist in the activation of bleaches, in the decomposition of organic material and the disinfection of both running and stored water.

Another preferable household appliance according to embodiments of the invention is a household appliance for storing, preparing or processing food and the self-cleaning catalytically active surface is an interior surface provided in the interior of the household appliance which comes into contact with food and/or food remains during operation of the household appliance.

The interior surface can thus be, for example, an inner wall of an oven, of a microwave or an inner wall and/or a shelf in a fridge.

In another preferred embodiment the interior surface is provided in a container for storing, preparing or processing food. Such a container can be, for example, the container of a food processor or blender or the drawer in a fridge or freezer.

In a preferred embodiment the household appliance is a fridge, a freezer, a fridge freezer, an oven, a stove, a microwave, a coffee or tea making machine or a food processor.

Generally, the at least one self-cleaning catalytically active surface may also be an external surface of one of the above household appliances, as for example the surface of a hob or an oven door.

The household appliance according to the invention can also be a vacuum cleaner or a device for the cleaning or maintenance of a floor, wherein the at least one ROS generator containing surfaces are especially in the area of filters and nonwovens and the anther as well as the device for holding the anther.

Further examples for household appliances in accordance with the present invention are flow-type heaters; vapour stations and steam irons; air humidifiers and air conditioning devices. In these cases, the disinfection of the surfaces as well as of the water to be heated is of importance. In general it is also here important that by means of the disinfection of the surfaces, the transmission of possibly harmful microorganisms or fungi can be avoided in case of skin contact.

In the case of kitchen hoods as a further example for a household appliance according to the present invention the disinfection of the surfaces, in particular of the meshes and of the filter mats, as well as the disinfecting treatment of the air is of importance.

Another important use is in surfaces of filter units. The self-cleaning surfaces as described herein can be the surfaces in fluff of dust filters.

The invention is moreover directed to a method for operating a household appliance with at least one self-cleaning catalytically active surface containing at least one reactive oxygen species (ROS) generator, wherein the self-cleaning catalytically active surface can come into contact with flowing or static water and/or food or food remains during operation of the household appliance and wherein the at least one ROS generator is selected from the group consisting of compounds with the formula (I), (II) or (III),

**Ag-B-M-Sulfur** **(I)**

wherein
B is selected from the group consisting of Al, Ga, In, Sn, TI, Pb and Bi; and
M is selected from the group consisting of Ti, Zr, Cr, Mo, Mn, Fe, Ru, Ni, Pd, Cu, Zn and Cd;

**MO_{q}-A** **(II)**

wherein
q = 2 or 3; and
M is selected from the group consisting of Zr, Ti, Cr, Mo, W and Mn; and
A is acetylacetonate or a derivative thereof selected from the group consisting of compounds with the formula: 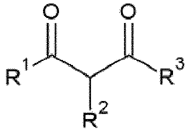 wherein
R¹, R² and R³ are independently selected from the group consisting of H, Cl, Br, I, and C₁ to C₈₀ hydrocarbon and polymer radicals;

**YSe-Rₓ-MO₂** **(III)**

wherein
Y is selected from Sn or Pb; and
Rₓ is a carbon allotrope; and
M is selected from the group consisting of Zr, Ti, Cr, Mo, W and Mn.

The invention has the advantage that an easy to clean household appliance, in particular a water-bearing household appliance which has a markedly lower susceptibility to dirt, particularly involving microorganisms is made available in a simple and cost-effective manner.

A particular advantage of the use of the ROS generators described herein in surfaces, in particular interior surfaces, lies therein that the lifetime of the ROS generator compounds described herein exceeds those of the known polyoxometalates. Said surfaces are able to function longer without any additional activation or addition energy usage being necessary. Only the use of an additional circulating element or air infeed element would require additional energy, although still slightly compared with other water-processing method such as membrane filtration or electrochemical processes.

Furthermore, the effectiveness of the catalytically active surfaces of the present invention exceeds those of polyoxometalate containing surfaces as the rate of reactive oxygen species generation is higher, sometimes significantly higher. In this way, a comparably high concentration of reactive oxygen species can be maintained also in long range distance to the self-cleaning catalytically active surface. Also with the ROS generators according to the invention no additional reagents need to be dosed in and activation of the catalysts, for example, by UV radiation can be dispensed with.

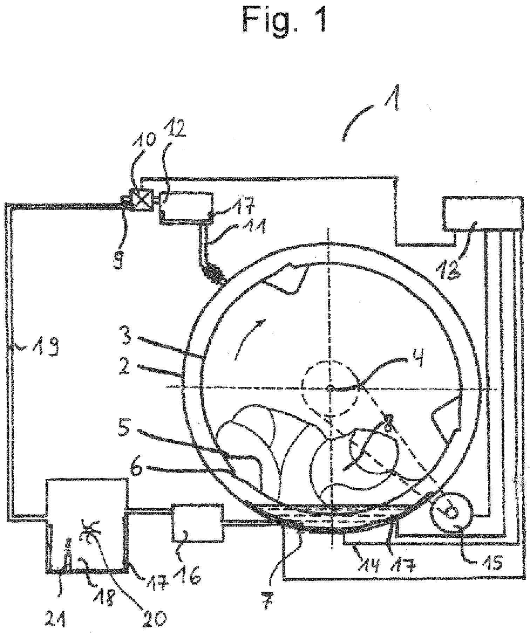

In the following the invention will be described in more detail by reference to Fig. 1.

Fig. 1 is a schematic representation of the present relevant parts of an embodiment of the household appliance according to the invention configured as a washing machine as non-limiting example.

The washing machine 1 of this embodiment has an outer tub 2 in which a drum 3 is rotatably mounted and can be driven by a drive motor 15. For ergonomic reasons, the rotation axis 4 of the drum 3 is oriented upwardly out of the horizontal by a small angle, so that easier access to, and inspection of the interior of the drum 3 is provided. With this arrangement, in cooperation with specially formed laundry agitators 5 and scooping devices 6 for the washing liquor 7 at the internal surface of the drum jacket, intensification of the flow of washing liquor 7 through the laundry items 8 can be achieved.

The washing machine 1 also has a water feed system which comprises a water connection fixture for the domestic water supply 9, an electrically controllable valve 10 and a feed pipe 11 which extends to the outer tub 2 and is fed via a detergent dispenser tray 12 from which the feed liquid is able to transport washing agent portions to the outer tub 2.

A heating device 14 is also provided in the outer tub 2. The valve 10 and the heating device 14 can be controlled by a program control system 12 depending on a program execution plan which can be linked to a time program and/or to the reaching of particular measured values of parameters such as the liquor level, the liquor temperature, the rotary speed of the drum 3, etc. within the washing machine 1.16 denotes a pump for the liquid, in particular washing liquor 7, in the outer tub 2.

A water reservoir 18 can store the greywater which has been used for rinsing the laundry item 8. Said greywater can be used for a later washing cycle. For this purpose, the water reservoir 18 is connected via a line ("feed pipe for rinsing liquid") 19 to the valve 10 which also regulates the fresh water feed. A circulating element 20 and an air infeed element 21, which can also be controlled by the program control system 13 are provided in the water reservoir 18. With these measures, a particularly efficient oxidation reaction is possible at the interior surface 17 in the water reservoir 18.

Interior surfaces 17 which contain Ag-In-Ni-Sulfur in the form of a nanocomposite, are applied in the detergent dispenser tray 12, in the outer tub 2 and in the water reservoir 18.

### Reference numerals

- 1: Household appliance, water-bearing household appliance, washing machine
- 2: (Outer) tub
- 3: Laundry drum
- 4: Rotation axis of the drum
- 5: Laundry agitators
- 6: Scooping devices
- 7: Washing liquor
- 8: Laundry items
- 9: Domestic water supply
- 10: Valve
- 11: Feed pipe
- 12: Detergent dispenser tray
- 13: Program control system
- 14: Heating device
- 15: Drive motor
- 16: Pump
- 17: Interior surface containing a ROS generator
- 18: Water reservoir
- 19: Feed pipe for rinsing water
- 20: Circulating element
- 21: Air infeed element

## Claims

1. A household appliance (1) with at least one self-cleaning catalytically active surface containing at least one reactive oxygen species (ROS) generator (17), **characterized in that** the at least one ROS generator is selected from the group consisting of compounds with the formula (I), (II) or (III);
**Ag-B-M-Sulfur** **(I)**
wherein
B is selected from the group consisting of Al, Ga, In, Sn, Tl, Pb and Bi; and
M is selected from the group consisting of Ti, Zr, Cr, Mo, Mn, Fe, Ru, Ni, Pd, Cu, Zn and Cd;
**MO_{q}-A** **(II)**
wherein
q = 2 or 3; and
M is selected from the group consisting of Zr, Ti, Cr, Mo, W and Mn; and
A is acetylacetonate or a derivative thereof selected from the group consisting of compounds with the formula: 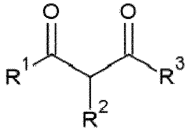 wherein
R¹, R² and R³ are independently selected from the group consisting of H, Cl, Br, I, and C₁ to C₈₀ hydrocarbon and polymer radicals;
**YSe-Rₓ-MO₂** **(III)**
wherein
Y is selected from Sn or Pb; and
Rₓ is a carbon allotrope; and
M is selected from the group consisting of Zr, Ti, Cr, Mo, W and Mn.

2. The household appliance (1) of claim 1, wherein the at least one ROS generator is
**ZrO₂-A** **(II')**
wherein A is acetylacetonate.

3. The household appliance (1) of claim 1, wherein the at least one ROS generator is
**Ag-in-Ni-Sulfur** **(I')**

4. The household appliance (1) of claim 1, wherein the at least one ROS generator is
**PbSe-Cx-TiO₂** **(III')**
wherein Cₓ is graphene.

5. The household appliance (1) of any of claims 1 to 4, wherein the self-cleaning catalytically active surface (17) is obtainable by admixing at least one ROS generator with the surface forming material prior to or during its manufacture.

6. The household appliance (1) of any of claims 1 to 4, wherein the self-cleaning catalytically active surface (17) is obtainable by generating a layer containing at least one ROS generator on the surface providing item.

7. The household appliance (1) of claim 6, wherein the self-cleaning catalytically active surface (17) has an at least one ROS generator containing upper layer with a thickness of from 0.01 to 0.5 mm.

8. The household appliance (1) of any of claims 1 to 7, wherein the household appliance (1) is a water-bearing household appliance (1) and the self-cleaning catalytically active surface (17) is an interior surface (17) provided in the interior of the household appliance (1) which comes into contact with flowing or static water during operation of the household appliance (1).

9. The household appliance (1) of claim 8, wherein the interior surface (17) is provided in a water reservoir.

10. The household appliance (1) of any of claims 1 to 9, wherein the household appliance (1) is a dishwasher or a laundry treatment device or a coffee or tea making machine.

11. The household appliance (1) of claim 10, wherein the household appliance (1) is a laundry treatment device belonging to the group consisting of a washing machine (1) and a washer-dryer.

12. The household appliance (1) of any of claims 1 to 7, wherein the household appliance (1) is a household appliance for storing, preparing or processing food and the self-cleaning catalytically active surface (17) is an interior surface (17) provided in the interior of the household appliance (1) which comes into contact with food and/or food remains during operation of the household appliance

13. The household appliance (1) of claim 12, wherein the interior surface (17) is provided in a container for storing, preparing or processing food.

14. The household appliance (1) of any claims 1 to 7, 12 or 13, wherein the household appliance (1) is a fridge, a freezer, a fridge freezer, an oven, a stove, a microwave, a coffee or tea making machine or a food processor.

15. A method for operating a household appliance (1) with at least one self-cleaning catalytically active surface containing at least one reactive oxygen species (ROS) generator (17), wherein the self-cleaning catalytically active surface (17) can come into contact with flowing or static water and/or food or food remains during operation of the household appliance (1) and wherein the at least one ROS generator is selected from the group consisting of compounds with the formula (I), (II) or (III),
**Ag-B-M-Sulfur** **(I)**
wherein
B is selected from the group consisting of Al, Ga, In, Sn, Tl, Pb and Bi; and
M is selected from the group consisting of Ti, Zr, Cr, Mo, Mn, Fe, Ru, Ni, Pd, Cu, Zn and Cd;
**MO_{q}-A** **(II)**
wherein
q = 2 or 3; and
M is selected from the group consisting of Zr, Ti, Cr, Mo, W and Mn; and
A is acetylacetonate or a derivative thereof selected from the group consisting of compounds with the formula: 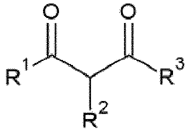 wherein
R¹, R² and R³ are independently selected from the group consisting of H, Cl, Br, I, and C₁ to C₈₀ hydrocarbon and polymer radicals.
**YSe-Rₓ-MO₂** **(III)**
wherein
Y is selected from Sn or Pb; and
Rₓ is a carbon allotrope; and
M is selected from the group consisting of Zr, Ti, Cr, Mo, W and Mn.
